## Europäisches Patentamt
## European Patent Office
## Office européen des brevets

(11) Numéro de publication: **0 253 744**
**A1**

(12) # DEMANDE DE BREVET EUROPEEN

(21) Numéro de dépôt: **87401679.3**

(22) Date de dépôt: **17.07.87**

(51) Int. Cl.⁴: **C 12 P 5/02**

(30) Priorité: **17.07.86 FR 8610431**

(43) Date de publication de la demande:
**20.01.88 Bulletin 88/03**

(84) Etats contractants désignés:
**AT BE CH DE ES GB GR IT LI LU NL SE**

(71) Demandeur: **GAZ DE FRANCE**
**23, rue Philibert Delorme**
**F-75017 Paris (FR)**

(72) Inventeur: **Belaich, Jean Pierre**
**33, rue Floralia Villa no 8**
**F-13009 Marseille (FR)**

**Fardeau, Marie Laure**
**Chemin de Bellepeire Les pennes Mirabeau**
**F-13170 (FR)**

**Peillex, Jean Paul**
**Le Lyautey C6 520, rue de St Hilaire**
**F-34000 Montpellier (FR)**

**Pavia, André**
**408, rue Valery Larbaud**
**F-34100 Montpellier (FR)**

(74) Mandataire: **Gillard, Marie-Louise et al**
**Cabinet Beau de Loménie 55, Rue d'Amsterdam**
**F-75008 Paris (FR)**

(54) **Procédé de production de méthane à rendement élevé par culture de Méthanobacterium thermoautotrophicum ou de toute autre bactérie méthanogène ayant les mêmes propriétés physiologiques de croissance.**

(57) La présente invention a pour objet un procédé de production de méthane avec rendement élevé. Ledit procédé consiste à cultiver sur un milieu de culture contenant une source d'azote et de sels assimilables la bactérie méthanogène Methanobacterium thermoautotrophicum ou toute autre bactérie méthanogène ayant les mêmes propriétés physiologiques de croissance, sous une alimentation forcée en mélange gazeux $H_2/CO_2$

EP 0 253 744 A1

**Description**

Procédé de production de méthane à rendement élevé par culture de Méthanobactérium thermoautotrophicum, ou de toute autre bactérie méthanogène ayant les mêmes propriétés physiologiques de croissance.

La présente invention concerne un procédé de production de méthane à rendement élevé par culture de Methanobacterium thermoautotrophicum ou de toute autre bactérie méthanogène ayant les mêmes propriétés physiologiques de croissance, dans des conditions particulières.

Il est bien connu que le méthane peut être produit par des bactéries dites méthanogènes. Parmi ces bactéries figurent les bactéries méthanogènes hydrogénophiles, qui sont capables de produire du méthane à partir d'un mélange gazeux $CO_2$-$H_2$. Certaines de ces bactéries sont également thermophiles et notamment Methanococcus thermolitotrophicus et Methanobacterium thermoautotrophicum.

Les propriétés physiologiques de la bactérie Methanococcus thermolithotrophicus et plus précisément l'énergétique de la croissance de cet organisme ont été décrits par Fardeau et Belaich das Arch. Microbiol. (1986) 114:381-385.

La bactérie Methanobacterium thermoautotrophicum a été étudiée par plusieurs équipes de recherche et est décrite notamment par:
- Schönheit et al. dans Arch. Microbiol. I27, 59-65 (1980);
- Seely et al. dans Biochem. Biophys. Rest. Comm. 116 : II25-II28 (1983);
- Brandis et al. dans Zbl. Bakt. Hyg. Abt. Grig. (2, 3II-3I7, (1981)), et
- Daniel et al. dans Bioeng. 14 : I99-2I3 (1984).

La plupart de ces chercheurs ont étudié cette bactérie en culture discontinue (ou culture en "batch"). Les conditions de culture préconisées par ces auteurs ne permettent pas l'obtention d'une productivité et d'un pourcentage élevés en méthane dans l'effluent gazeux du fermenteur.

D'autre part, des travaux réalisés par le demandeur ont montré qu'il était possible d'atteindre avec les bactéries méthanogènes thermophiles des activités spécifiques de production de méthane très importantes (372 $m^3$ de méthane par jour et par kg, poids sec, de bactéries) mais qu'il fallait trouver une solution biotechnologique ou biologique permettant d'accroître le pourcentage de méthane dans l'effluent et la productivité du réacteur par augmentation de la biomasse active. En effet, malgré l'importante activité spécifique des bactéries méthanogènes thermophiles, la productivité des fermenteurs était très faible, de l'ordre de 30 V/V/J. Il en est de même pour les biomasses maximum, obtenues en milieu non renouvelé, qui ne dépassaient pas, pour Méthanococcus thermolitotrophicus (MTL), la valeur de 0,8 g poids sec/litre. Les résultats de productivité étaient qualitativement identiques pour la deuxième bactérie utilisée: Methanobacterium thermoautotrophicum (MTA) bien que la biomasse obtenue dans le fermenteur ait été plus élevée (5 g/l)/.

Jusqu'à présent, il était donc impensable d'envisager la production de méthane à l'échelle industrielle par culture de l'une ou de l'autre des bactéries citées précédemment.

On a maintenant trouvé que l'on peut obtenir du méthane avec aussi bien une productivité élevée qu'un haut rendement en méthane dans l'effluent produit, par culture en continu de Methanobacterium thermoautotrophicum ou de toute autre bactérie méthanogène ayant les mêmes propriétés physiologiques de croissance sous alimentation forcée de gaz, c'est-à-dire avec une vitesse élevée de transfert de gaz.

Ainsi la présente invention a pour objet un procédé de production de méthane à rendement élevé qui consiste à cultiver les bactéries ci-dessus en continu sur un milieu de culture adéquat et sous alimentation forcée en gaz $H_2$/$CO_2$. Ainsi en utilisant une vitesse de transfert de gaz d'au moins 60 litres de mélange $CO_2$/$H_2$ par litre et par heure, au moins 96 % du gaz entrant dans le fermenteur est transformé en méthane.

La vitesse de transfert de gaz est déterminée à partir de la productivité en méthane multipliée par cinq.

Aux fins de l'invention, cette vitesse doit être d'au moins 50 litres par litre et par heure.

La culture de Methanobacterium thermoautotrophicum ou de toute autre bactérie méthanogène ayant les mêmes propriétés physiologiques de croissance est réalisée dans un fermenteur sur un milieu de culture contenant essentiellement une source d'azote et une source de sels assimilables.

Un exemple de composition du milieu de culture approprié aux fins de l'invention est donné ci-après:
$KH_2PO_4$ 50 mM
NaCl 40 mM
$NH_4Cl$ 50 mM
$Na_2CO_3$ 19 mM
Titriplex I 0,5 mM
$MgCl_2$ 0,2 mM
$FeCl_2$ 50 µM
$COCl_2$ 1 µM
$Na_2MoO_4$ 1 µM
$Ni(NO_3)_2$, 6 $H_2O$ 5 µM
cysteine chlorhyddrate 2,86 mM
$Na_2S$, 9 $H_2O$ 2,1 mM

Un tel milieu doit être préparé en anaérobiose et stérilisé par exemple pendant environ 20 minutes à environ 110° C.

Le procédé de l'invention est mis en oeuvre à la température à laquelle se multiplie la bactérie, par exemple pour Methanobacterium thermoautotrophicum, à une température de 65° C environ.

Le fermenteur est alimenté en substrat constitué d'hydrogène et de gaz carbonique dans un rapport approprié, par exemple dans un rapport $H_2$/$CO_2$ de 80/20 %.

L'alimentation en substrat gazeux du fermenteur est avantageusement effectuée par un diffuseur en verre fritté ou tout autre matériau poreux de faible porosité donnant une répartition de taille des bulles de gaz très faible par rapport au volume liquide et qui est placé en dessous de la turbine d'agitation nécessaire à la dispersion de ces bulles et à la turbulence du milieu.

La mesure des capacités de transfert de l'ensemble turbine diffiseur peut être réalisée en suivant l'oxydation d'une solution de sulfite par l'oxygène de l'air. Le coefficient de transfert de gaz mesuré par cette méthode appelé $K_L.a$, atteint une valeur de $3000 h^{-1}$ à 65°C pour 60 litre/litre/heure d'air à l'entrée et pour une vitesse d'agitation de 600 tours par minute alors que sa valeur maximale pour les fermenteurs équipés d'une couronne de diffusion classique ne dépasse pas $1500 h^{-1}$ dans les mêmes conditions.

Il est maintenant possible, grâce au procédé de l'invention d'obtenir un rendement élevé en $CH_4$, c'est-à-dire une productivité d'au moins 250 V/V/J de méthane avec un pourcentage d'au moins 85 % dans l'effluent.

L'invention va être maintenant décrite en détail par l'exemple non limitatif ci-après :

EXEMPLE :
On a utilisé un fermenteur Interscience "Labo 2000" sur lequel a ajouté deux pipes coudées en verre fritté (porosité 0) entre la turbine et le coupleur magnétique d'entraînement de la turbine.

Le fermenteur a été utilisé au maximum de sa puissance de chauffe sans le système de refroidissement.

L'agitation étant réglable de 300 à 1200 tours par minute, les meilleurs résultats ont été obtenus à partir de 900 tours par minute.

Le volume de travail était de 1 litre.

On a utilisé le milieu de culture défini précédemment. Ce milieu de culture liquide stocké dans un ballon de 20 l en anaérobiose (sous courant $H_2/CO_2$ continu) a été amené au fermenteur à l'aide d'une pompe péristaltique ("Masterflex") dans des tuyaux de silicone épais ("Victoria").Le débit d'alimentation en gaz a été commandé par une régulation électronique adaptée au système. Le substrat était consituté d'un mélange gazeux $H_2/CO_2$ dans un rapport $80/20 \pm 0,4 \%$ ("Prodair").

Le niveau liquide dans le fermenteur a été réglé par un tube affleurant la surface et commun à la sortie des gaz et du milieu, une légère surpression du gaz entraînant l'évacuation du liquide excédentaire jusqu'à affleurement du tube. L'effluent a été récolté dans un ballon stock stérile et en anaérobiose.

Les flux de gaz entrant et sortant ont été mesurés à l'aide de débimètres.

Le pH et le potentiel redox ont été mesurés régulièrement mais non régulés.

Les gaz de sortie ont été réfrigérés pour condenser la vapeur d'eau et analysés par chromatographie en phase gazeuse.

Les résultats obtenus avec Methanobacterium thermoautotrophicum sont reportés sur les figures 1 à 3 annexées, lesquelles représentent respectivement.

- figure 1: la productivité en $CH_4$ (ordonnées de droite: V/V/J) et en biomasse (ordonnées à gauche ; g/l) de Methanobacterium thermoautotrophicum en fonction de la vitesse d'agitation à taux de dilution et débit de $CO_2/H_2$ constants à l'entrée ; les pourcentages de $CH_4$ dans l'effluent sont indiqués sur la courbe ;

- figure 2 : la productivité en biomasse (g/l) de Methanobacterium thermoautotrophicum en fonction du débit d'entrée du mélange gazeux à taux de dilution et vitesse d'agitation constants ;

- figure 3 : la productivité en $CH_4$ (V/V/J) de Methanobacterium thermoautotrophicum en fonction du débit d'entrée du mélange gazeux à taux de dilution et vitesse d'agitation constants ; les pourcentages de $CH_4$ dans l'effluent sont indiqués sur la courbe.

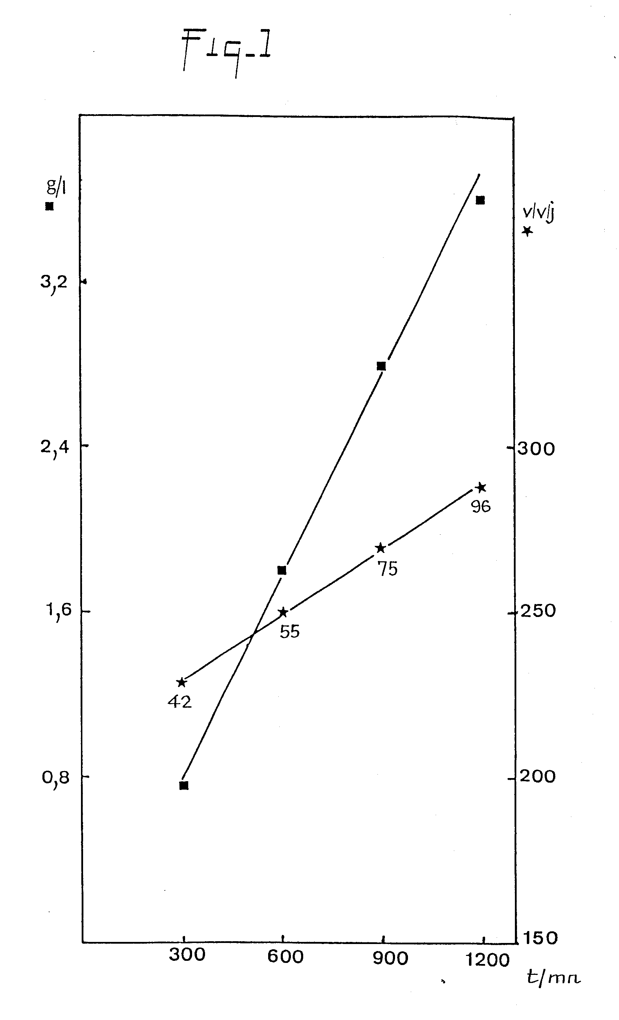

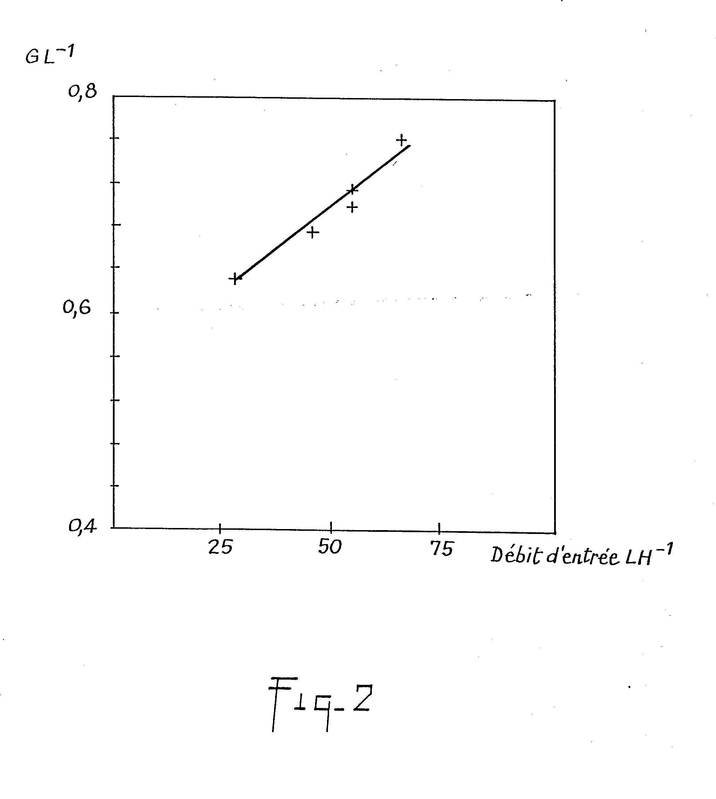

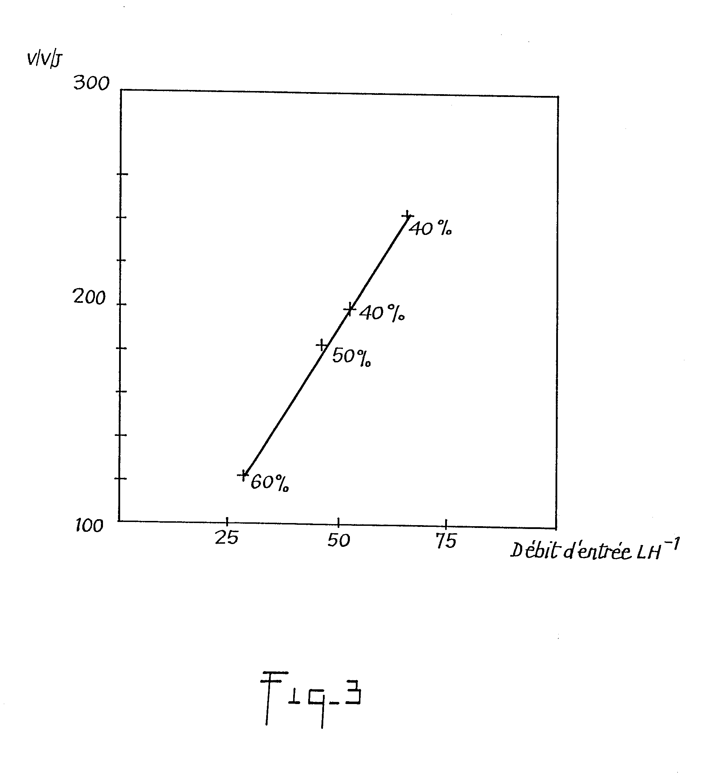

La figure 1 montre clairement que la biomasse, à l'état stationnaire, du fermenteur est proportionnelle à la vitesse de rotation de la turbine. La culture de MTA passe d'une concentration cellulaire de 0,8 g/l à 3,6 g/l ; dans le meme temps, la productivité du réacteur dépasse de 228 à 288 V/V/J et le pourcentage de méthane dans l'effluent gazeux de 42 à 96 % . Les figures 2 et 3 montrent que pour un taux de dilution et une vitesse d'agitation constants la biomasse et la productivité du réacteur sont proportionnelles au débit du gaz d'alimentation de la culture (mélange $H_2/CO_2$). Le meilleur couple (pourcentage de $CH_4$ dans l'effluent , productivité du réacteur) obtenu a été de 96 % de $CH_4$ et 288 V/V/J pour une vitesse de rotation de la turbine de 1200 tours par minute, le débit d'entrée étant de 60,5 l/h ; le débit de sortie de 12,5 l/heure ; et la biomasse de 3,6 g/l poids sec.

Ces résultats sont d'autant plus surprenants et originaux que des expériences analogues réalisées avec Méthanococcus thermolitotrophicus n'ont pas permis d'atteindre la même productivité en $CH_4$. Les productivités maximums obtenues dans ce cas sont toujours inférieures à 80 V/V/J avec un pourcentage de méthane dans l'effluent de 50 % environ.

L'ensemble de ces essais montre que le procédé de l'invention résulte à la fois de la sélection des bactéries méthanogènes particulières ayant des propriétés physiologiques de croissance spécifiques et de conditions spécifiques de culture.

**Revendications**

1. Procédé de production de méthane avec rendement élevé, caractérisé en ce qu'il consiste à cultiver sur un milieu de culture contenant une source d'azote et de sels assimilables la bactérie méthanogène Methanobacterium thermoautotrophicum ou toute autre bactérie méthanogène ayant les mêmes propriétés physiologiques de croissance, sous une alimentation forcée en mélange gazeux $H_2/CO_2$.

2. Procédé selon la revendication 1, caractérisé en ce que l'alimentation forcée en mélange gazeux est obtenue avec une vitesse de transfert de gaz d'au moins 50 litres par litre et par heure

0253744

Fig-1

Fig-2

0253744

Fig-3

Office européen
des brevets

**RAPPORT DE RECHERCHE EUROPEENNE**

Numéro de la demande

EP 87 40 1679

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| X | GB-A- 721 823 (MATEPA)<br>* Revendications 1-3 *<br><br>--- | 1,2 | C 12 P 5/02 |
| X | CHEMICAL ABSTRACTS, vol. 84, no. 25, 21 juin 1976, page 418, résumé no. 178192m, Columbus, Ohio, US; K. RAJAGOPALAN: "Methane fermentation of rubber (Hevea brasiliensis) latex effluent", & CAN. J. MICROBIOL. 1976, 22(3), 342-6<br>* Résumé *<br><br>--- | 1,2 | |
| X | GB-A-2 107 735 (MATSUSHITA)<br>* Revendications 1-5 *<br><br>--- | 1,2 | |
| X | SOVIET INVENTIONS ILLUSTRATED, section chemical, semaine K 48, résumé no. 831668, P16E17, 18 janvier 1984, Derwent Publications Ltd, Londres, GB; & SU-A-992 569 (BIOCHEM. INST. BAKH.) 30-01-1983<br>* En entier *<br><br>--- | 1 | DOMAINES TECHNIQUES RECHERCHES (Int. Cl.4)<br><br>C 12 P |
| A | ARCHIVES OF BIOCHEMISTRY, vol. 14, no. 1,2, juillet 1947, pages 57-70, US; A.J. KLUYVER et al.: "On the fermentation of carbon monoxide by pure cultures of methane bacteria"<br>* Page 57, dernier alinéa *<br><br>----- | 1 | |

Le présent rapport de recherche a été établi pour toutes les revendications

| Lieu de la recherche | Date d'achèvement de la recherche | Examinateur |
|---|---|---|
| LA HAYE | 12-10-1987 | WIESER, M.R.J. |